Europäisches Patentamt

European Patent Office

Office européen des brevets

(11) Publication number: **0 355 779**
**A2**

# EUROPEAN PATENT APPLICATION

(21) Application number: 89115425.4

(22) Date of filing: 22.08.89

(51) Int. Cl.4: **C07K 3/18** , **C12N 9/60** , **C12N 15/57**

Claims for the following Contracting State: ES.

(30) Priority: 26.08.88 US 236957
13.09.88 US 243735

(43) Date of publication of application:
28.02.90 Bulletin 90/09

(84) Designated Contracting States:
AT BE CH DE ES FR GB GR IT LI LU NL SE

(71) Applicant: THE GENERAL HOSPITAL CORPORATION
55 Fruit Street
Boston MA 02114(US)

(72) Inventor: Smith, John A.
19 Thatcher Street 5
Brooklene Massachusetts 02146(US)
Inventor: Chang, Yie-Hwa
91 Winslow Avenue
Sommerville Massachusetts 02143(US)

(74) Representative: Klein, Otto, Dr. et al
Hoechst AG Zentrale Patentabteilung
Postfach 80 03 20
D-6230 Frankfurt am Main 80(DE)

(54) ·Isolation, purification, and characterization of the methionine-specific aminopeptidases: MAS I and MAS X.

(57) This invention is directed toward methionine-specific aminopeptidase enzymes, and specifically toward a methionine-specific aminopeptidase produced via natural or recombinant methods. The invention is further directed to a method for purifying and using the methionine-specific aminopeptidase.

EP 0 355 779 A2

# ISOLATION, PURIFICATION, AND CHARACTERIZATION OF THE METHIONINE-SPECIFIC AMINOPEP-TIDASES: MAS I AND MAS X

## Background of the Invention

Protein synthesis is always initiated with either methionine or N-formylmethionine (Ben-Bassat et al., J. Bacteriol. 169: 751-757 (1987); Sherman, F. et al., BioEssays 3:27-31 (1985); Capecchi, M.R., Biochem. 55:1517-1524 (1966)); which references are incorporated herein by reference. Deformylase enzymes catalyze the removal of the N-formyl group thus leaving methionine as the amino-terminal residue of natural proteins (Adams, J.M. J. Molec. Biol. 33:571-589 (1968)). In most proteins, enzyme mediated, post-translational cleavage of the peptide bonds has been observed. Such processing may include cleavage of a signal secretory or localization peptide, an N-terminal acetylated residue, the specific cleavage of the amino terminal methionine residue, etc. (Ben-Bassat et al., J. Bacteriol. 169:751-757 (1987)).

Enzymes which are capable of cleaving a peptide bond between the N-terminal most and the penultimate residues of a protein or peptide are known as "aminopeptidases." Such enzymes have been identified in a variety of microorganisms, such as E. coli, Bacillus licheniformis, Bacillus subtilus, Salmonella typhimurium and Mycoplasma salivarium (Carter, T.H. et al., J. Bacteriol. 159:453-459 (1984); Hayashi, S., J. Biol. Chem. 259:10448-10454 (1984); Hermsdorf, C.L., Biochem. 17:3370-3376 (1978); McHugh, G.L. et al., J. Bacteriol 120:364-371 (1974); Miller, C.G. et al., J. Bacteriol. 135:603-611 (1978), Simmonds, S., Biochem. 9:1-8 (1970); and Shibata, K. et al., J. Bacteriol. 169:3409-3413 (1987)).

Yeast, and in particular, the yeast Saccharomyces cerevisiae, have also been found to contain aminopeptidases. Trumbly, R.J. et al. (J. Bacteriol. 156:36-48 (1983)), for example, identified a yeast leucine aminopeptidase which was capable of cleaving a leucine-containing peptide. Yeast peptidases are reviewed by Achstetter, T. et al. (Yeast 1:139-157 (1985)), which reference is herein incorporated by reference.

The specific cleavage of an amino-terminal methionine residue is catalyzed by a class of enzymes termed "methionine-specific aminopeptidases." Such enzymes are reviewed by Ben-Bassat et al., who reported the identification of an E. coli methionine-specific aminopeptidase (J. Bacteriol. 169:751-757 (1987)), and by Sherman, F. et al., who provide a theoretical discussion of the properties of such enzymes (BioEssays 3:27-31 (1985)).

As discussed above, although all proteins are initially synthesized with an amino-terminal methionine residue, this residue is frequently removed by an endogenous methionine-specific aminopeptidase. The failure to remove the amino-terminal methionine can significantly alter the properties of the protein. For example, recombinant human growth hormone which contains an amino-terminal methionine elicits an immune response in approximately 30% recipients. In contrast, the removal of the amino-terminal methionine results in a less immunogenic molecule. Additionally, the retention of an amino-terminal methionine residue may alter the solubility, biologic half-life, biologic activity, conformation, etc. of a protein. These considerations are of special concern to the field of recombinant DNA technology, since proteins produced in accordance with the methods of this field may often contain an amino-terminal methionine residue which would have been removed by a methionine-specific aminopeptidase if the protein had been naturally produced.

Thus, a methionine-specific aminopeptidase capable of cleaving not only naturally produced produced proteins but also proteins produced through recombinant DNA methods would be highly desirable.

## Summary of the Invention

According to the present invention, methionine-specific aminopeptidases capable of cleaving the peptide bond between the carboxyl group of a methionine residue and the amino group of an adjacent amino acid residue were isolated and substantially purified. The present invention therefore relates to the purification of the enzymes, to the uses for the enzymes, and to the substantially purified enzymes themselves.

In detail, the invention provides a substantially purified methionine-specific aminopeptidase enzyme having an ability to cleave the peptide bond between an amino-terminal methionine residue, and every penultimate amino-terminal amino acid residue selected from the group consisting of: Ala, Asp, Glu, Gln, Gly, Ile, Leu, Lys, Met, Phe, Ser and Thr.

EP 0 355 779 A2

The invention also includes a substantially purified methionine-specific aminopeptidase enzyme having an ability to cleave the peptide bond between an amino-terminal methionine residue, and every penultimate amino-terminal amino acid residue selected from the group consisting of: Val, Ala, Asp, Glu, Gln, Gly, His, Pro, Ser and Thr.

The invention also includes a substantially purified substrate-specific aminopeptidase enzyme having an ability to cleave the peptide bond between an amino-terminal residue X, and a penultimate Ile residue of a peptide, wherein X is an amino-terminal amino acid residue selected from the group consisting of: Leu, Phe and Trp.

The invention especially pertains to the above methionine-specific aminopeptidases which are MAS I and MAS X.

The invention also includes a recombinant DNA molecule comprising a genetic sequence coding for either of the above methionine-specific aminopeptidases.

The invention also pertains to a method for recovering substantially purified methionine-specific aminopeptidase from a sample containing the aminopeptidase comprising the following steps:

(a) recovering crude methionine-specific amino-peptidase from a sample containing the aminopeptidase;

(b) subjecting the crude methionine-specific amino-peptidase from step (a) to ion exchange chromatography; and

(c) recovering any methionine-specific aminopeptidase purified by the ion exchange chromatography.

The invention further pertains to the above-described method which additionally comprises the steps of:

(d) subjecting the recovered methionine-specific aminopeptidase of step (c) to hydroxylapatite chromatography; and

(e) recovering any methionine-specific aminopeptidase purified by the hydroxylapatite chromatography.

The invention further pertains to the above-described method which additionally comprises the steps of:

(f) subjecting the recovered methionine-specific aminopeptidase of step (e) to additional ion exchange chromatography; and

(g) recovering any methionine-specific aminopeptidase purified by the additional ion exchange chromatography.

The invention also provides a method for removing an amino-terminal methionine residue from a peptide containing the amino-terminal residue, and containing as a penultimate amino-terminal amino acid residue, an amino acid selected from the group consisting of Ala, Asp, Glu, Gln, Gly, Ile, Leu, Lys, Met, Phe, Ser and Thr, which method comprises incubating the peptide in the presence of the methionine-specific aminopeptidase of MAS I to thereby remove the amino-terminal residue.

The invention also provides a method for removing an amino-terminal methionine residue from a peptide containing the amino-terminal residue, and containing as a penultimate amino-terminal amino acid residue, an amino acid selected from the group consisting of: Val, Ala, Asp, Glu, Gln, Gly, His, Pro, Ser and Thr, which method comprises incubating the peptide in the presence of the methionine-specific aminopeptidase of MAS X to thereby remove the amino-terminal residue.

The invention also includes the embodiment of the above-described method wherein the methionine-specific aminopeptidase is immobilized to a solid support.

Detailed Description of the Invention

I. Isolation, and Purification of the Methionine-specific Aminopeptidase of the Present Invention

In accordance with the present invention, either of the methionine-specific aminopeptidases of the present invention can be isolated from a sample containing the enzyme. Methionine-specific aminopeptidase appears to be ubiquitous, being found in all eukaryotes, including plants, animals, and multicellular organisms. A different enzyme having methionine-specific aminopeptidase activity is also believed to be present in prokaryotes. Therefore, any cell source is contemplated in this invention. As used herein, the sample containing either of the enzymes will be referred to simply as "sample." Unless specified as being unique to one or the other of the methionine-specific aminopeptidases of the present invention, the recombinant DNA methods described herein can be employed to clone and express either or both of the aminopeptidases of the present invention.

3

In the following description of the purification of the methionine-specific aminopeptidase of the present invention, the activity of the aminopeptidase may be assayed by any convenient method. The preferred assay for methionine-specific aminopeptidase activity is as follows:

Enzyme activity can be assayed using the peptide, Met-Ala-Ser, as the substrate. In such an assay, an aliquot of 10 $\mu$l from each fraction collected from a chromatographic column is added to a labeled well of a microtiter plate containing 45-$\mu$l of the solution which contains 4 mM Met-Ala-Ser, 10 mM HEPES-K$^+$, pH 7.35, 0.2 mM $CoCl_2$, 0.2 M KCl, 1.5 mM $MgCl_2$, 0.02% $NaN_3$, 10 $\mu$g of L-amino acid oxidase, 115 $\mu$g of horseradish peroxidase and 10 $\mu$g of o-dianisidine dihydrochloride. The reactions are carried out at room temperature, for one hour, and the absorbance at 450 nm, or equivalently at 414 nm, is recorded.

The isolation and purification of the methionine-specific aminopeptidases of the present invention will hereinafter be described from a yeast sample, although it is to be understood that other samples could be used as the source material.

Yeast cells, preferably Saccharomyces cerevisiae, are grown in suitable medium (preferably YPD medium: 1% yeast extract, 2% Bacto-peptone, 2% glucose) to an $A_{600}$ of 5-20. Preparations of cells are grown to late-log (i.e. to an $A_{600}$ of 18-20) or, more preferably, to mid-log (i.e. to an $A_{600}$ of 7-9). Although both such preparations are found to have methionine-specific aminopeptidase activity, preparations of mid-log cells are preferred since the MAS I purified from such preparations lacks a 70,000 d contaminant which is found to co-purify with the MAS I obtained from late-log cells. Cells are harvested, preferably by centrifugation, and resuspended in YPD medium containing 15% glycerol. The cells may then be frozen to -70 $^\circ$C, and stored for an extended period.

Frozen cells are thawed, preferably by incubation in a 30 $^\circ$C water bath, and collected by centrifugation at room temperature. The pelleted cells are resuspended in a suitable buffer such as Buffer S (1 M Sorbitol, 50 mM Tris, pH 7.8, 10 mM $MgCl_2$, 3 mM DTT), supplemented with lyticase, and incubated at 30 $^\circ$C, to permit spheroplast formation. The spheroplasts are then collected, washed in additional Buffer S, lysed and homogenized in hypotonic buffer, such as buffer A (10 mM HEPES-K$^+$, pH 7.0, 1.5 mM $MgCl_2$, 10 mM KCl, and 0.5 mM DTT). Homogenization is preferably accomplished at 4 $^\circ$C using a Dounce homogenizer. Yeast methionine-specific aminopeptidase is released into the cellular lysate by gentle shaking. After removing any debris by centrifugation, glycerol is added to the supernatant of the lysate (10% final concentration) in order to increase the stability of the methionine-specific aminopeptidase.

The above-described supernatant of the cellular lysate is then concentrated to 100 ml by the use of a PM-10 ultrafiltration membrane and dialyzed against a suitable buffer, such as Buffer H (10 mM HEPES-K$^+$, pH 7.35, 1.5 mM $MgCl_2$, 0.5 mM DTT, 0.2% $NaN_3$, 10% glycerol), supplemented to contain 0.05 M KCl. Residual cell biomaterials in the supernatant are removed to avoid protein-biomaterial aggregation in subsequent steps. Ion exchange chromatography can be used for this procedure. In this step, the ion exchange used is preferably CM-Sepharose chromatography, most preferably employing CM-Sepharose CL-6B with 0.05 M KCl. The column is preferably pre-equilibrated with two column volumes of the same buffer used for dialysis. The concentrated supernatant is then applied to the column and eluted with a linear gradient of 0.05 M (250 ml) to 0.5 M (250 ml) KCl in buffer H at a rate of between 15-20 ml/hr. Fractions, preferably of 2-10 ml, and most preferably of approximately 5 ml, are collected. The fractions are numbered consecutively with the first collected fraction being designated as "fraction 1."

Methionine-specific aminopeptidase activity is typically found in fractions 42 through 46, and 57 through 67. These two sets of fractions are separately pooled to produce two methionine-specific aminopeptidase-containing preparations. The methionine-specific aminopeptidase in the preparation formed by pooling fractions 42-46 (average conductivity ~ 6.7 ms) is designated "MAS X," the methionine-specific aminopeptidase in the preparation formed by pooling fractions 57-67 (average conductivity ~ 11.1 ms) is designated "MAS I." The MAS I and MAS X preparations are preferably concentrated using PM-10 ultrafiltration membranes.

The preparation containing MAS I may be further purified using hydroxylapatite chromatography. The concentrated MAS I preparation is dialyzed against a suitable phosphate buffer (such as, for example, PP Buffer which contains 10 mM potassium phosphate buffer, pH 7.15, 0.5 mM DTT, 50 mM KCl, 10% glycerol, 0.02% $NaN_3$) and applied to a hydroxylapatite column that has previously been equilibrated against the PP Buffer. The applied material is eluted from the column with a linear gradient of 0.01 M (250 ml) to 0.5 M PP Buffer. Fractions of between 4-5 ml are collected and assayed for methionine-specific aminopeptidase activity. Fractions having such activity (typically fractions 95-105, of average conductivity ~ 27.1 ms), are pooled and concentrated to a volume of 5 ml, using a PM-10 ultrafiltration membrane. The pooled MAS I fractions are preferably further purified using a second CM-Sepharose CL-6B column.

The concentrated eluate from the above-described hydroxylapatite chromatography are dialyzed against Buffer H (supplemented to contain 0.05 M KCl), and applied to a CM-Sepharose column (preferably, CM-Sepharose CL-6B). The applied material is then eluted isocratically, or, more preferably, through the use of a linear gradient of 0.05 M (250 ml) to 0.5 M (250 ml) KCl in Buffer H at a rate of approximately 15-20 ml/hr. Fractions of approximately 4 ml are collected, assayed for methionine-specific aminopeptidase activity, and fractions having such activity are pooled. Typically, fractions 103 - 111 (average conductivity ~ 14.0 ms) contained such activity. Fractions having methionine-specific aminopeptidase activity may be concentrated using PM-10 ultrafiltration membranes.

Using this series of chromatography steps, yeast methionine-specific aminopeptidase MAS I was purified to near homogeneity from cells harvested in mid-log phase (i.e. from cells at an $A_{600}$ of approximately 8). SDS-polyacrylamide gels of the purified sample reveal that MAS I migrates as a single band of about apparent molecular weight 41,000 when stained with Coomassie blue. The electrophoresis was performed according to the method of Laemmli, U.K. (Nature 227:680-685 (1970)) using a 10% gel. The gel was stained with Coomassie blue. Standard proteins of 45, 66, 97, 116, and 205 kd molecular weight were used to calibrate the gel. If MAS I is purified from cells harvested in late-log phase (i.e. from cells at an $A_{600}$ of 18-20), the final preparation is found to have only MAS I and a single contaminant of apparent molecular weight of 70,000.

The terms peptide, polypeptide, and protein are all intended to refer to polymers of amino acid residues. A peptide is a polymer of at least two amino acid residues joined to one another by a peptide bond. A polypeptide is a polymer of several amino acid residues joined to one another by a peptide bond. A protein is a large polymer of many amino acid residues joined to one another by a peptide bond.

The term "aminopeptidase" as used herein is intended to refer to an enzyme capable of attacking and cleaving the peptide bond which joins the amino-terminal amino acid residue of a peptide (and thus also of a polypeptide or protein) to the penultimate amino-terminal amino acid residue of that peptide (or polypeptide or protein).

The aminopeptidases of the present invention are termed "methionine-specific aminopeptidases" or "substrate-specific aminopeptidases." An aminopeptidase is said to be "methionine-specific" if (1) it is capable of cleaving an amino-terminal methionine residue from a peptide or protein, and (2) peptides or proteins which possess an amino-terminal methionine residue are the preferred or exclusive substrate of the aminopeptidase. An aminopeptidase is said to be "substrate-specific" if only certain peptides or proteins (i.e. not every peptide or protein) can serve as a substrate for the enzyme. Such suitable substrates possess particular amino-terminal and penultimate amino-terminal residues, which are joined by a peptide bond. The substrate specificity of the aminopeptidase is defined by its ability or inability to cleave a particular substrate. The substrate specificity of the aminopeptidases of the present invention is defined below.

The present invention concerns an aminopeptidase enzyme which is "substantially pure" or which has been "substantially purified." As used herein, the terms "substantially pure" or "substantially purified" are intended to be equivalent, and to describe an aminopeptidase which is substantially free of a compound normally associated with the enzyme in its natural state, i.e., a protein, carbohydrate, lipid, etc. The term is further meant to describe an aminopeptidase which is homogeneous by one or more of the assays of purity or homogeneity used by those of skill in the art. For example, a substantially pure aminopeptidase will show constant and reproducible characteristics within standard experimental deviations for parameters such as the following: molecular weight, chromatographic techniques, etc. The term " substantially pure," however, is not meant to exclude artificial or synthetic mixtures of the enzymes with other compounds. The term is also not meant to exclude the presence of impurities which do not interfere with the biological activity of the enzyme, and which may be present, for example, due to incomplete purification.

II. Characterization of the Methionine-specific Aminopeptidases MAS I and MAS X of the Present Invention

Molecular Weight of MAS I

SDS-polyacrylamide gels of the purified sample reveal that MAS I migrates as a single band of about apparent molecular weight 41,000 when stained with Coomassie blue. The electrophoresis was performed according to the method of Laemmli, U.K. (Nature 227:680-685 (1970)) using a 10% gel. The gel was stained with Coomassie blue. Standard proteins of 45, 66, 97, 116, and 205 kd molecular weight were used to calibrate the gel. The apparent molecular weight of MAS X has not been determined.

Substrate-Specificity

A partial determination of substrate specificity of MAS I and MAS X was carried out using a set of thirty-six pure peptides. The peptides had either of two sequences:

(I)     MET-X-ILE-PRO-GLU-THR

or (II)     X-ILE-PRO-GLU-THR

wherein "X" refers to one of the following eighteen amino acids: Val, Ala, Arg, Asp, Cys, Glu, Gln, Gly, His, Ile, Leu, Lys, Met, Phe, Pro, Ser, Thr and Trp.

MAS I was found to be able to cleave those peptides of group I wherein "X" was either Ala, Asp, Glu, Gln, Gly, Ile, Leu, Lys, Met, Phe, Ser or Thr. No other tested peptide of group I could be cleaved by MAS I. MAS I was able to cleave only the Met-containing peptide of group II; it was unable to cleave any other group II peptide tested.

MAS X was found to be able to cleave those peptides of group I wherein "X" was either Val, Ala, Asp, Glu, Gln, Gly, His, Pro, Ser, or Thr. No other tested peptide of group I could be cleaved. MAS X was able to cleave those peptides of group II wherein "X" was either Leu, Met, Phe, or Trp. No other peptide of group II tested could be cleaved.

III. Genetic Engineering of MAS I and MAS X

The identification of fractions which contain substantially purified MAS I or MAS X activity permits the amino acid sequence of these enzymes to be determined, and further permits these molecules to be produced through the application of recombinant DNA techniques. Thus, the present invention includes not only substantially purified MAS I and MAS X, and methods for their use, but also includes the amino acid sequences of these enzymes, the genetic sequences coding for these enzymes, vehicles containing such genetic sequences, hosts transformed therewith, enzyme production by transformed host expression, and utilization of the enzymes in the cleavage of methionine-containing peptides or proteins. MAS I is the preferred methionine-specific aminopeptidase of the present invention.

In order to obtain the amino acid sequence of the enzymes, the enzymes in the substantially purified fractions are recovered as by electroelution from a polyacrylamide gel. The recovered molecules may then be sequenced, preferably using an automated sequenator, and the amino acid sequence of the enzyme thereby determined.

Alternatively, and more preferably, the amino acid sequence of the enzymes is determined through an analysis of the DNA or, more preferably, the cDNA sequence, of the gene which encodes the enzyme. In order to obtain these nucleic acid sequences, a source which contains the MAS I or MAS X gene or cDNA sequence is screened with oligonucleotide probes which encode fragments of the MAS I or MAS X enzyme.

In order to prepare the oligonucleotide probes, the substantially purified enzyme is recovered and fragmented as with cyanogen bromide, or with proteases such as papain, chymotrypsin, trypsin, etc. (Oike, Y., et al., J. Biol. Chem. 257:9751-9758 (1982); Liu, C., et al., Int. J. Pept. Protein Res. 21:209-215 (1983)). The resulting peptides are separated, preferably by reverse-phase HPLC, and subjected to amino acid sequencing. To accomplish this task, the peptides are preferably analyzed by automated sequenators.

Once one or more suitable peptide fragments have been sequenced, the DNA sequences capable of encoding them are examined. If the peptides are greater than 10 amino acids long, this sequence information is generally sufficient to permit one to clone a gene sequence such as those which encode the enzymes of the present invention. Because the genetic code is degenerate, however, more than one codon may be used to encode a particular amino acid (Watson, J.D., In: Molecular Biology of the Gene, 3rd Ed., W.A. Benjamin, Inc., Menlo Park, CA (1977), pp. 356-357). Using the genetic code, one or more different oligonucleotides can be identified, each of which would be capable of encoding the enzyme peptides. The probability that a particular oligonucleotide will, in fact, constitute the actual enzyme fragment-encoding sequence can be estimated by considering abnormal base pairing relationships and the frequency with which a particular codon is actually used (to encode a particular amino acid) in eukaryotic cells. Such "codon usage rules" are disclosed by Lathe, R., et al., J. Molec. Biol. 183:1-12 (1985). Using the "codon usage rules" of Lathe, a single oligonucleotide, or a set of oligonucleotides, that contains a theoretical "most probable" nucleotide sequence capable of encoding the enzyme fragment's peptide sequence is identified.

Techniques for synthesizing oligonucleotides are disclosed by, for example, Wu, R., et al., Prog. Nucl. Acid. Res. Molec. Biol. 21:101-141 (1978)). Procedures for constructing recombinant molecules in accordance with the above-described method are disclosed by Maniatis, T., et al., In: Molecular Cloning, A Laboratory Manual, Cold Spring Harbor Press, Cold Spring Harbor, NY (1984), which reference is herein incorporated by reference.

Although occasionally an amino acid sequence may be encoded by only a single oligonucleotide, frequently the amino acid sequence may be encoded by any of a set of similar oligonucleotides. Importantly, whereas all of the members of this set contain oligonucleotides which are capable of encoding the peptide fragment and, thus, potentially contain the same oligonucleotide sequence as the gene which encodes the peptide fragment, only one member of the set contains the nucleotide sequence that is identical to the nucleotide sequence of the gene. Because this member is present within the set, and is capable of hybridizing to DNA even in the presence of the other members of the set, it is possible to employ the unfractionated set of oligonucleotides in the same manner in which one would employ a single oligonucleotide to clone the gene that encodes the peptide.

The oligonucleotide, or set of oligonucleotides, containing the theoretical "most probable" sequence capable of encoding the enzyme fragment peptide is used to identify the sequence of a complementary oligonucleotide or set of oligonucleotides which is capable of hybridizing to the "most probable" sequence, or set of sequences. An oligonucleotide containing such a complementary sequence can be employed as a probe to identify and isolate a gene sequence which encodes the enzyme (Maniatis, T., et al., Molecular Cloning, A Laboratory Manual, Cold Spring Harbor Press, Cold Spring Harbor, NY (1982)).

The DNA probe may be labeled with a detectable group. Such detectable group can be any material having a detectable physical or chemical property. Such materials have been well-developed in the field of immunoassays and in general most any label useful in such methods can be applied to the present invention. Particularly useful are enzymatically active groups, such as enzymes (see Clin. Chem. 22:1243 (1976)), enzyme substrates (see British Pat. Spec. 1,548,741), coenzymes (see U.S. Pat. Nos. 4,230,797 and 4,238,565) and enzyme inhibitors (see U.S. Pat. No. 4,134,792); fluorescers (see Clin. Chem. 25:353 (1979)); chromophores; luminescers such as chemiluminescers and bioluminescers (see Clin. Chem. 25:512 (1979)); specifically bindable ligands; proximal interacting pairs; and radioisotopes such as $^{3}H$, $^{35}S$, $^{32}P$, $^{125}I$ and $^{14}C$. Such labels and labeling pairs are detected on the basis of their own physical properties (e.g., fluorescers, chromophores and radioisotopes) or their reactive or binding properties (e.g., enzymes, substrates, coenzymes and inhibitors). For example, a cofactor-labeled probe can be detected by adding the enzyme for which the label is a cofactor and a substrate for the enzyme. For example, one can use an enzyme which acts upon a substrate to generate a product with a measurable physical property. Examples of the latter include, but are not limited to, beta-galactosidase, alkaline phosphatase and peroxidase.

A suitable oligonucleotide, or set of oligonucleotides, which is capable of encoding a fragment of the gene sequence which encodes the enzyme (or which is complementary to such an oligonucleotide, or set of oligonucleotides) is identified (using the above-described procedure), synthesized, and hybridized by means well known in the art, against a DNA or, more preferably, a cDNA preparation derived from cells which are capable of expressing the enzyme. Single-stranded oligonucleotide molecules complementary to the "most probable" enzyme peptide encoding sequences can be synthesized using procedures which are well known to those of ordinary skill in the art (Belagaje, R., et al., J. Biol. Chem. 254:5765-5780 (1979); Maniatis, T., et al., In: Molecular Mechanisms in the Control of Gene Expression, Nierlich, D.P., et al., Eds., Acad. Press, NY (1976); Wu, R., et al., Prog. Nucl. Acid Res. Molec. Biol. 21:101-141 (1978); Khorana, R.G., Science 203:614-625 (1979)). Additionally, DNA synthesis may be achieved through the use of automated synthesizers. Techniques of nucleic acid hybridization are disclosed by Maniatis, T., et al. (In: Molecular Cloning, A Laboratory Manual, Cold Spring Harbor Laboratories, Cold Spring Harbor, NY (1982)), and by Haymes, B.D., et al. (In: Nucleic Acid Hybridization, A Practical Approach, IRL Press, Washington, DC (1985)), which references are herein incorporated by reference.

A DNA sequence coding for MAS I or MAS X may be derived from a variety of sources. For example, mRNA coding for either enzyme may be isolated from the tissues of any species that produces the enzyme, and identified, by using the Northern blot method (Alwine et al., Method Enzymol. 68:220-242 (1979)) and labeled oligonucleotide probes. The mRNA may then be converted to cDNA by techniques known to those skilled in the art. The probes may be synthesized based on the known amino acid sequence of MAS I as described above. Alternatively, genomic DNA may be isolated and employed. The source of DNA or cDNA used will preferably have been enriched for the gene sequence which encodes the enzyme. Such enrichment can most easily be obtained from cDNA obtained by extracting RNA from cells, such as yeast cells, which produce high levels of the enzyme. An example of such a cell is a Saccharomyces cerevisiae cell.

Any of a variety of methods may be used to clone a gene sequence which encodes the enzymes of the present invention. One such method entails analyzing a shuttle vector library of cDNA inserts (derived from a cell that expresses the enzymes of the present invention) for the presence of an insert which contains a gene sequence which is capable of encoding the enzyme. Such an analysis may be conducted by transfecting cells with the vector, and then assaying for enzyme expression.

To identify and clone the gene sequence capable of encoding either of the enzymes of the present invention, a DNA, or more preferably a cDNA, library is screened for its ability to hybridize with the oligonucleotide probes described above. Suitable DNA preparations (such as human genomic DNA) are enzymatically cleaved, or randomly sheared, and ligated into recombinant vectors. The ability of these recombinant vectors to hybridize to the above-described oligonucleotide probes is then measured. Vectors found capable of such hybridization are then analyzed to determine the extent and nature of the enzyme sequences which they contain. Based purely on statistical considerations, a gene sequence capable of encoding any of the enzymes of the present invention could be unambiguously identified (via hybridization screening) using an oligonucleotide probe having only 18 nucleotides.

In an alternative way of cloning a gene sequence which encodes the enzymes of the present invention, a library of expression vectors is prepared by cloning DNA or, more preferably, cDNA (from a cell capable of expressing the enzyme) into an expression vector. The library is then screened for members capable of expressing a protein which binds to enzyme-specific antibody, and which has a nucleotide sequence that is capable of encoding polypeptides that have the same amino acid sequence as the enzyme, or fragments thereof. In this embodiment, DNA, or more preferably cDNA, is extracted and purified from a cell which is capable of expressing the enzyme. The purified cDNA is fragmented (by shearing, endonuclease digestion, etc.) to produce a pool of DNA or cDNA fragments. DNA or cDNA fragments from this pool are then cloned into an expression vector in order to produce a genomic or cDNA library of expression vectors whose members each contain a unique cloned DNA or cDNA fragment.

Thus, in summary, the identification of the methionine-specific aminopeptidases of the present invention permits the identification of a theoretical "most probable" DNA sequence, or a set of such sequences, capable of encoding peptide sequences of these enzymes. By constructing an oligonucleotide complementary to this theoretical sequence (or by constructing a set of oligonucleotides complementary to the set of "most probable" oligonucleotides), one obtains a DNA molecule (or set of DNA molecules), capable of functioning as a probe to identify and isolate a gene sequence capable of encoding either of the enzymes of the present invention.

Techniques such as, or similar to, those described above have successfully enabled the cloning of genes for human aldehyde dehydrogenases (Hsu, L.C., et al., Proc. Natl. Acad. Sci. USA 82:3771-3775 (1985)), fibronectin (Suzuki, S., et al., Eur. Mol. Biol. Organ. J. 4:2519-2524 (1985)), the human estrogen receptor gene (Walter, P., et al., Proc. Natl. Acad. Sci. USA 82:7889-7893 (1985)), tissue-type plasminogen activator (Pennica, D., et al., Nature 301:214-221 (1983)) and human term placental alkaline phosphatase complementary DNA (Kam, W., et al., Proc. Natl. Acad. Sci. USA 82:8715-8719 (1985)).

IV. Expression of the Methionine-Specific Aminopeptidase Encoding Sequences

DNA or cDNA molecules which encode the MAS I or MAS X enzyme can be operably linked into an expression vector and introduced into a host cell to enable the expression of the MAS I or MAS X enzyme by that cell. Two DNA sequences (such as a promoter region sequence and a desired enzyme encoding sequence) are said to be operably linked if the nature of the linkage between the two DNA sequences does not (1) result in the introduction of a frame-shift mutation, (2) interfere with the ability of the promoter region sequence to direct the transcription of the desired enzyme encoding gene sequence, or (3) interfere with the ability of the desired enzyme gene sequence to be transcribed by the promoter region sequence.

A DNA sequence encoding MAS I may be recombined with vector DNA in accordance with conventional techniques, including blunt-ended or stagger-ended termini for ligation, restriction enzyme digestion to provide appropriate termini, filling in of cohesive ends as appropriate, alkaline phosphatase treatment to avoid undesirable joining, and ligation with appropriate ligases.

The present invention encompasses the expression of the desired enzyme in either prokaryotic or eukaryotic cells. Preferred eukaryotic hosts include yeast, fungi (especially Aspergillus), mammalian cells (such as, for example, human or primate cells) either in vivo, or in tissue culture. Yeast are the preferred hosts of the present invention.

The use of yeast provides substantial advantages in that yeast can also carry out post-translational peptide modifications including glycosylation. A number of recombinant DNA strategies exist which utilize strong promoter sequences and high copy number of plasmids which can be utilized for production of the desired proteins in yeast. Yeast recognize leader sequences on cloned mammalian gene products and secrete peptides bearing leader sequences (i.e., pre-peptides). Mammalian cells provide post-translational modifications to protein molecules including correct folding or glycosylation at correct sites. Mammalian cells which may be useful as hosts include cells of fibroblast origin such as VERO or CHO-K1, and their derivatives. For a mammalian host, several possible vector systems are available for the expression of the desired enzyme. A wide variety of transcriptional and translational regulatory sequences may be employed, depending upon the nature of the host. The transcriptional and translational regulatory signals may be derived from viral sources, such as adenovirus, bovine papilloma virus, simian virus, or the like, where the regulatory signals are associated with a particular gene which has a high level of expression. Alternatively, promoters from mammalian expression products, such as actin, collagen, myosin, etc., may be employed. Transcriptional initiation regulatory signals may be selected which allow for repression or activation, so that expression of the genes can be modulated. Of interest are regulatory signals which are temperature-sensitive so that by varying the temperature, expression can be repressed or initiated, or are subject to chemical regulation, e.g., metabolite.

The expression of the desired enzyme in eukaryotic hosts requires the use of eukaryotic regulatory regions. Such regions will, in general, include a promoter region sufficient to direct the initiation of RNA synthesis. Preferred eukaryotic promoters include the promoter of the mouse metallothionein I gene (Hamer, D., et al., J. Mol. Appl. Gen. 1:273-288 (1982)); the TK promoter of Herpes virus (McKnight, S., Cell 31:355-365 (1982)); the SV40 early promoter (Benoist, C., et al., Nature (London) 290:304-310 (1981)); the yeast gal4 gene promoter (Johnston, S.A., et al., Proc. Natl. Acad. Sci. (USA) 79:6971-6975 (1982); Silver, P.A., et al., Proc. Natl. Acad. Sci. (USA) 81:5951-5955 (1984)).

As is widely known, translation of eukaryotic mRNA is initiated at the codon which encodes the first methionine. For this reason, it is preferable to ensure that the linkage between a eukaryotic promoter and a DNA sequence which encodes the desired enzyme (or a functional derivative thereof) does not contain any intervening codons which are capable of encoding a methionine (i.e., AUG). The presence of such codons results either in the formation of a fusion protein (if the AUG codon is in the same reading frame as the desired enzyme encoding DNA sequence) or a frame-shift mutation (if the AUG codon is not in the same reading frame as the desired enzyme encoding sequence).

Preferred prokaryotic hosts include bacteria such as E. coli, Bacillus, Streptomyces, Pseudomonas, Salmonella, Serratia, etc. The most preferred prokaryotic host is E. coli. Bacterial hosts of particular interest include E. coli K12 strain 294 (ATCC 31446), E. coli X1776 (ATCC 31537), E. coli W3110 (F⁻, lambda⁻, prototrophic (ATCC 27325)), other enterobacteria (such as Salmonella typhimurium or Serratia marcescens), and various Pseudomonas species. The prokaryotic host must be compatible with the replicon and control sequences in the expression plasmid.

To express the desired enzyme (or a functional derivative thereof) is a prokaryotic cell (such as, for example, E. coli, B. subtilis, Pseudomonas, Streptomyces, etc.), it is necessary to operably link the desired enzyme encoding sequence to a functional prokaryotic promoter. Such promoters may be either constitutive or, more preferably, regulatable (i.e., inducible or derepressible). Examples of constitutive promoters include the int promoter of bacteriophage λ, and the bla promoter of the β-lactamase gene of pBR322, etc. Examples of inducible prokaryotic promoters include the major left and right promoters of bacteriophage λ - ($P_L$ and $P_R$), the trp, recA, lacZ, lacI, gal, and tac promoters of E. coli, the α-amylase (Ulmanen, I., et al., J. Bacteriol. 162:176-182 (1985)), the σ-28-specific promoters of B. subtilis (Gilman, M.Z., et al., Gene 32:11-20 (1984)), the promoters of the bacteriophages of Bacillus (Gryczan, T.J., In: The Molecular Biology of the Bacilli, Academic Press, Inc., NY (1982)), and Streptomyces promoters (Ward, J.M., et al., Mol. Gen. Genet. 203:468-478 (1986)). Prokaryotic promoters are reviewed by Glick, B.R., (J. Ind. Microbiol. 1:277-282 (1987)); Cenatiempo, Y. (Biochimie 68:505-516 (1986)); and Gottesman, S. (Ann. Rev. Genet. 18:415-442 (1984)).

Proper expression in a prokaryotic cell also requires the presence of a ribosome binding site upstream from the gene-encoding sequence. Such ribosome binding sites are disclosed, for example, by Gold, L., et al. (Ann. Rev. Microbiol. 35:365-404 (1981)).

The desired enzyme encoding sequence and an operably linked promoter may be introduced into a recipient prokaryotic or eukaryotic cell either as a non-replicating DNA (or RNA) molecule, which may either be a linear molecule or, more preferably, a closed covalent circular molecule. Since such molecules are incapable of autonomous replication, the expression of the desired enzyme may occur through the transient expression of the introduced sequence. Alternatively, permanent expression may occur through the integration of the introduced sequence into the host chromosome.

In one embodiment, a vector is employed which is capable of integrating the desired gene sequences into the host cell chromosome. Cells which have stably integrated the introduced DNA into their chromosomes can be selected by also introducing one or more markers which allow for selection of host cells which contain the expression vector. The marker may complement an auxotrophy in the host (such as leu2, or ura3, which are common yeast auxotrophic markers), biocide resistance, e.g., antibiotics, or heavy metals;. such as copper or the like. The selectable marker gene can either be directly linked to the DNA gene sequences to be expressed, or introduced into the same cell by co-transfection.

In a preferred embodiment, the introduced sequence will be incorporated into a plasmid or viral vector capable of autonomous replication in the recipient host. Any of a wide variety of vectors may be employed for this purpose. Factors of importance in selecting a particular plasmid or viral vector include: the ease with which recipient cells that contain the vector may be recognized and selected from those recipient cells which do not contain the vector; the number of copies of the vector which are desired in a particular host; and whether it is desirable to be able to "shuttle" the vector between host cells of different species.

Any of a series of yeast gene expression systems can be utilized. Examples of such expression vectors include the yeast 2-micron circle, the expression plasmids YEP13, YCP and YRP, etc., or their derivatives. Such plasmids are well known in the art (Botstein, D., et al., Miami Wntr. Symp. 19:265-274 (1982); Broach, J.R., In: The Molecular Biology of the Yeast Saccharomyces: Life Cycle and Inheritance, Cold Spring Harbor Laboratory, Cold Spring Harbor, NY, p. 445-470 (1981); Broach, J.R., Cell 28:203-204 (1982)). YEP13 is the preferred vector of the present invention.

For a mammalian host, several possible vector systems are available for expression. One class of vectors utilize DNA elements which provide autonomously replicating extra-chromosomal plasmids, derived from animal viruses such as bovine papilloma virus, polyoma virus, adenovirus, or SV40 virus. A second class of vectors relies upon the integration of the desired gene sequences into the host chromosome. Cells which have stably integrated the introduced DNA into their chromosomes may be selected by also introducing one or more markers which allow selection of host cells which contain the expression vector. The marker may provide for prototropy to an auxotrophic host, biocide resistance, e.g., antibiotics, or heavy metals, such as copper or the like. The selectable marker gene can either be directly linked to the DNA sequences to be expressed, or introduced into the same cell by co-transformation. Additional elements may also be needed for optimal synthesis of mRNA. These elements may include splice signals, as well as transcription promoters, enhancers, and termination signals. The cDNA expression vectors incorporating such elements include those described by Okayama, H., Mol. Cell. Biol. 3:280 (1983), and others.

Preferred prokaryotic vectors include plasmids such as those capable of replication in E. coli (such as, for example, pBR322, ColE1, pSC101, pACYC 184, πVX. Such plasmids are, for example, disclosed by Maniatis, T., et al. (In: Molecular Cloning, A Laboratory Manual, Cold Spring Harbor Press, Cold Spring Harbor, NY (1982)). Bacillus plasmids include pC194, pC221, pT127, etc. Such plasmids are disclosed by Gryczan, T. (In: The Molecular Biology of the Bacilli, Academic Press, NY (1982), pp. 307-329). Suitable Streptomyces plasmids include pIJ101 (Kendall, K.J., et al., J. Bacteriol. 169:4177-4183 (1987)), and Streptomyces bacteriophages such as φC31 (Chater, K.F., et al., In: Sixth International Symposium on Actinomycetales Biology, Akademiai Kaido, Budapest, Hungary (1986), pp. 45-54). Pseudomonas plasmids are reviewed by John, J.F., et al. (Rev. Infect. Dis. 8:693-704 (1986)), and Izaki, K. (Jpn. J. Bacteriol. 33:729-742 (1978)).

Once the vector or DNA sequence containing the constructs has been prepared for expression, the DNA constructs may be introduced into an appropriate host. Various techniques may be employed, such as protoplast fusion, calcium phosphate precipitation, electroporation or other conventional techniques. After the fusion, the cells are grown in media and screened for appropriate activities. Expression of the desired sequence results in the production of the methionine-specific amino-peptidase.

The methionine-specific aminopeptidase of the invention may be isolated and purified from the above-described recombinant molecules in accordance with conventional methods, such as extraction, precipitation, chromatography, affinity chromatography, electrophoresis, or the like.

V. Uses of Methionine-Specific Aminopeptidase

Any of the methionine-specific aminopeptidases of the present invention, once produced and purified, can be used, for example, in a pharmaceutical, or manufacturing, environment to remove the amino-terminal methionine from peptides and proteins for which such removal is desired in order to improve the stability, solubility, biologic activity, biologic half-life, etc. or to decrease the immungenicity of the peptide or protein. It can, in addition, be used to cleave proteins, and polypeptides in accordance with its substrate specificities.

The methionine-specific aminopeptidase may be incubated with a desired substrate as a soluble enzyme, or may be immobilized, by means well known in the art, onto a solid support (such as, for example, glass, latex, plastic, cellulose, etc.)

Having now generally described this invention, the same will be better understood by reference to

10

specific examples, which are included herein for purposes of illustration only, and are not intended to be limiting unless otherwise specified.

## EXAMPLE 1

### CELL GROWTH AND PREPARATION OF CRUDE EXTRACT

#### Cell Growth and Storage

Yeast strain TD 71.8 (alpha, ino3, leu2, his3, lys2) was grown at 30 °C in 1 liter YPD medium (1% yeast extract, 2% Bacto-peptone, 2% glucose) to late-log phase ($A_{600}$ ~18-20) or mid-log phase ($A_{600}$ ~8). The cells were harvested by centrifugation at 3,500 rpm for 10 min at 4 °C. The pellet (~ 25 g) was resuspended in 25 ml YPD medium containing 15% glycerol and stored at -70 °C.

#### Preparation of Whole Cell Extracts

Frozen yeast cells (~500 g wet weight) were thawed in a 30 °C water bath and collected by centrifugation at 3,500 rpm for 10 minutes at room temperature. The pellet was resuspended in 1 liter of buffer S (1 M sorbitol, 50 mM Tris, pH 7.8, 10 mM $MgCl_2$, 3 mM DTT) containing 50 mg lyticase. This mixture was incubated at 30 °C for 2 hours with shaking to induce spheroplast formation. Spheroplasts were collected by centrifugation at 3,500 rpm for 10 minutes at 4° and washed by gentle resuspension 1 liter of cold buffer S, collected by centrifugation at 3,500 rpm for 10 minutes and resuspended gently in 500 ml of buffer A (10 mM HEPES-K$^+$, pH 7.0, 1.5 mM $MgCl_2$, 10 mM KCl and 0.5 mM DTT). All the subsequent steps were carried out at 4 °C. The spheroplasts were lysed in this hypotonic buffer by 12-15 stokes with a tight-fitting pestle followed by 15-20 strokes with a loose - fitting pestel in a Dounce homogenizer and then the final KCl concentration was adjusted to 0.2 M by adding cold 2M KCl solution. The lysate was stirred on ice for 30 minutes, and the debris was removed by centrifugation at 17,000 rpm for 30 minutes. Glycerol was added to 10% in all the buffers used in subsequent purification procedures to additionally stabilize the aminopeptidase.

The fractions were assayed for methionine-specific amino-peptidase activity as described above.

## EXAMPLE 2

### PURIFICATION OF THE METHIONINE-SPECIFIC AMINOPEPTIDASE

#### CM-Sepharose CL-6B Chromatography

The crude lysate was concentrated to 100 ml by the use of a PM-10 ultrafiltration membrane and dialized 3 times against 2 liters of buffer H (10 mM HEPES-K$^+$, pH 7.35, 1.5 mM $MgCl_2$, 0.5 mM DTT, 0.2% $NaN_3$, 10% glycerol) supplemented to contain 0.05 M KCl. It was then loaded on a CM-Sepharose CL-6B column (2.5 x 45 cm) which had been equilibrated with the same buffer as that used for dialysis. This column was washed with 2 liters of buffer H containing 0.05 M KCl and then eluted with a linear gradient of 0.05 M (250 ml) to 0.5 M (250 ml) KCl in buffer H at 17 ml/hr. Fractions (5.0 ml) were collected; the fractions 42 to 46 and 57 to 67 which contain amnopeptidase activity were pooled respectively. Each of these pooled fractions was concentrated to a volume of 5 ml using a PM-10 ultrafiltration membrane. The aminopeptidase in fractions 57 to 67 was designated as "MAS I;" the other one in fractions 42 to 46 was designated as "MAX X." The MAS I was further purified by a hydroxylapatite column.

## Hydroxylapatite Chromatography

The concentrated eluate from the above CM-Sepharose CL-6B chromatography was dialyzed overnight 3 times against 1 liter of 0.01 M potassium phosphate buffer, pH 7.15, 0.5 mM DTT, 50 mM KCl, 10% glycerol, 0.02% NaN₃, and applied to a hydroxylapatite column (2.5 x 20 cm) equilibrated with the same buffer as that used for dialysis. The column was eluted with a linear gradient of 0.01 M (250 ml) to 0.5 M (250 ml) potassium phosphate buffer, pH 7.15, 0.5 mM DTT, 50 mM KCl, 10% glycerol, 0.02% NaN₃ at 18.6 ml/hr. Fractions (4.4 ml) were collected and the fractions containing aminopeptidase activity were pooled and concentrated to a volume of 5 ml, using a PM-10 ultrafiltration membrane. This aminopeptidase (MAS I) was further purified by a second CM-Sepharose CL-6B column.

## Second CM-Sepharose CL-6B Chromatography

The concentrated eluate from hydroxylapatite chromatography was dialyzed 3 times against 1 liter of buffer H containing 0.05 M KCl and applied to a CM-Sepharose CL-6B column (2.5 x 45 cm) which had been equilibrated with the same buffer as that used for dialysis. The column was eluted with a liner gradient of 0.05 M (250 ml) to 0.5 M (250 ml) KCl in buffer H at 18.9 ml/hr. Fractions (3.78 ml) were collected, and the fractions containing aminopeptidase activity were pooled and concentrated to 5 ml using a PM-10 ultrafiltration membrane.

The profiles of the fractions containing methionine-specific aminopeptidase activity are shown in Table 1.

Table 1

| Profile of Activity Containing Fractions | | | | |
|---|---|---|---|---|
| Column | Activity Data | | Protein Concentration 280 nm* | Average Conductivity of Active Fractions (ms) |
| | Fraction Numbers | Absorbance 450 nm* | | |
| MAS I | | | | |
| CM-Sepharose | 57-67 | 0.363 | 2.73 | 11.1 |
| Hydroxylapatite | 95-105 | 0.188** | 0.467 | 27.1 |
| CM-Sepharose | 103-111 | 0.056** | 0.273 | 14.0 |
| MAS X | | | | |
| CM-Sepharose | 42-46 | 0.333 | 1.994 | 6.7 |
| Hydroxylapatite | not done | -- | -- | -- |
| CM-Sepharose | not done | -- | -- | -- |

* Absorbance is average of pooled active fractions
** Absorbance is at 414 nm

Preparations of MAS I obtained from late-log cells and purified in the manner described above, were found to contain a single contaminant of approximately 70,000. In contrast, this contaminant was not present in preparations of MAS I obtained from mid-log cells which had been purified as described above. The 70,000 contaminant can be removed from the preparation by gel electroelution, gel filtration, or other conventional means.

## EXAMPLE 3

12

## ESTIMATION OF THE MOLECULAR WEIGHT OF AMINOPEPTIDASE BY SDS-POLYACRYLAMIDE GEL ELECTROPHORESIS

Electrophoresis was performed according to the method of Laemmli, U.K. (Nature 227:680-685 (1970)) using an 10% gel. The gel was stained with Coomassie blue, and the apparent molecular weight of the denatured MAS I was calculated from the relative mobility of protein standards including (1) myosin (205,000), (2) E. Coli beta-galactosidase (116,000), (3) rabbit muscle phosphorylase (97,000), (4) bovine serum albumin (66,000) and (5) egg albumin (45,000), and (6) carbonic anhydrase (29,000). The apparent molecular weight of MAS I was approximately 41,000. The apparent molecular weight of MAS X has not been determined.

### EXAMPLE 4

### SUBSTRATE SPECIFICITY

Thirty-six peptides were made to study the substrate specificity of the purified aminopeptidases of the present invention. Eighteen of the peptides had different N-terminal amino acid residues, and the remaining eighteen peptides had different amino acid residues at the penultimate amino-terminal position. The protein solution (10 $\mu$l) to be assayed was added to 90 $\mu$l of the solution containing 4 mM M-X-I-P-E-T or X-I-P-E-T, 10 mM HEPES-K$^+$, pH 7.35, 0.2 mM $CoCl_2$, 0.2 M KCl, 1.5 mM $MgCl_2$, 0.02% $NaN_3$, 30 $\mu$g of L-amino acid oxidase, 3 $\mu$g of horseradish peroxidase and 20 $\mu$g of o-dianisidine dihydrochloride. All the reactions were carried out at room temperature on a 96-well microtiter plate, and absorbance detected at 414 or 450 nm. Rapid color development indicates the presence of high peptidase acitivity on that specific peptide. The results of these experiments are shown in Tables 2 and 3. The identity of X is designated by the commonly used single letter abbreviations for the common amino acids: V = Val, A = Ala, R = Arg, D = Asp, C = Cys, E = Glu, Q = Gln, G = Gly, H = His, I = Ile, L = Leu, K = Lys, M = Met, F = Phe, P = Pro, S = Ser, T = Thr, and W = Trp. The numbers in Tables 2 and 3 represent qualitative estimtes of activity: 0 (no activity); 1 (+/-); 2 (+); 3 (+ +/-); 4 (+ +); 5 (+ + +/-); and 6 (+ + +).

### Table 2
### Substrate Specificity: M-X-I-P-E-T

| Identity of X: | V | A | R | D | C | E | Q | G | H | I | L | K | M | F | P | S | T | W |
|---|---|---|---|---|---|---|---|---|---|---|---|---|---|---|---|---|---|---|
| ENZYME: MAS I | 0 | 2 | 0 | 1 | 0 | 1 | 1 | 1 | 0 | 2 | 1 | 1 | 1 | 1 | 0 | 2 | 1 | 0 |
| MAS X | 1 | 6 | 0 | 2 | 0 | 1 | 1 | 1 | 1 | 0 | 0 | 0 | 0 | 0 | 6 | 4 | 1 | 0 |

### Table 3
### Substrate Specificity: X-I-P-E-T

| Identity of X: | V | A | R | D | C | E | Q | G | H | I | L | K | M | F | P | S | T | W |
|---|---|---|---|---|---|---|---|---|---|---|---|---|---|---|---|---|---|---|
| ENZYME: MAS I | 0 | 0 | 0 | 0 | 0 | 0 | 0 | 0 | 0 | 0 | 0 | 0 | 1 | 0 | 0 | 0 | 0 | 0 |
| MAS X | 0 | 0 | 0 | 0 | 0 | 0 | 0 | 0 | 0 | 0 | 1 | 0 | 1 | 1 | 0 | 0 | 0 | 1 |

Although the foregoing refers to particular preferred embodiments, it will be understood that the present invention is not so limited. It will occur to those ordinarily skilled in the art that various modifications may be made to the disclosed embodiments and that such modifications are intended to be within the scope of the present invention.

## Claims

1. A method for recovering substantially purified methionine-specific aminopeptidase from a sample containing said aminopeptidase,characterized by the following steps:

(a) recovering crude methionine-specific aminopeptidase from a sample containing said aminopeptidase;

(b) subjecting said crude methionine-specific aminopeptidase from step (a) to ion exchange chromatography; and

(c) recovering any methionine-specific aminopeptidase purified by said ion exchange chromatography, and, preferably,

(d) subjecting said recovered methionine-specific aminopeptidase of step (c) to hydroxylapatite chromatography; and

(e) recovering any methionine-specific aminopeptidase purified by said hydroxylapatite chromatography, and, especially,

(f) subjecting said recovered methionine-specific aminopeptidase of step (e) to additional ion exchange chromatography; and

(g) recovering any methionine-specific aminopeptidase purified by said additional ion exchange chromatography.

2. The method of claim 1,characterized in that said sample is isolated from yeast growing at mid-log or late-log phase.

3. The substantially purified methionine-specific aminopeptidase enzyme MAS I having an ability to cleave the peptide bond between an amino-terminal methionine residue, and every penultimate amino-terminal amino acid residue selected from the group consisting of: Ala, Asp, Glu, Gln, Gly, Ile, Leu, Lys, Met, Phe, Ser and Thr.

4. The substantially purified methionine-specific aminopeptidase enzyme MAS X having an ability to cleave the peptide bond between an amino-terminal methionine residue, and every penultimate amino-terminal amino acid residue selected from the group consisting of: Val, Ala, Asp, Glu, Gln, Gly, His, Pro, Ser and Thr, and having an ability to cleave the peptide bond between an amino-terminal residue X, and a penultimate Ile residue of a peptide, wherein X is an amino-terminal amino acid residue selected from the group consisting of: Leu, Phe and Trp.

5. A recombinant DNA molecule characterized by a genetic sequence coding for the methionine-specific aminopeptidase of claims 3 or 4.

6. A process for the production of the methionine-specific aminopeptidases MAS I and MAS X, characterized by expressing a recombinant DNA molecule of claim 5 in a suitable host.

7. A method for removing an amino-terminal methionine residue from a peptide containing said amino-terminal residue, and containing as a penultimate amino-terminal amino acid residue, an amino acid selected from the group consisting of Ala, Asp, Glu, Gln, Gly, Ile, Leu, Lys, Met, Phe, Ser and Thr, characterized by incubating said peptide in the presence of the methionine-specific aminopeptidase MAS I to thereby remove said amino-terminal residue.

8. A method for removing an amino-terminal methionine residue from a peptide containing said amino-terminal residue, and containing as a penultimate amino-terminal amino acid residue, an amino acid selected from the group consisting of: Val, Ala, Asp, Glu, Gln, Gly, His, Pro, Ser and Thr, characterized by incubating said peptide in the presence of the methionine-specific aminopeptidase MAS X to thereby remove said amino-terminal residue.

9. A method for removing an amino-terminal residue X selected from the group consisting of: Leu, Phe and Trp from a peptide containing said aminoterminal residue, and containing as a penultimate amino-terminal amino residue Ile, characterized by incubating said peptide in the presence of the aminopeptidase MAS X to thereby remove said amino-terminal residue X.

10. The method of claim 7, 8 or 9, characterized in that said methionine-specific aminopeptidase is immobilized to a solid support.

CLAIMS for the following contracting state:ES

1. A method for recovering substantially purified methionine-specific amnopeptidase from a sample containing said aminopeptidase,characterized by the following steps:

(a) recovering crude methionine-specific aminopeptidase from a sample containing said aminopeptidase;

(b) subjecting said crude methionine-specific aminopeptidase from step (a) to ion exchange chromatography; and

(c) recovering any methionine-specific aminopeptidase purified by said ion exchange chromatography, and, preferably,

(d) subjecting said recovered methionine-specific aminopeptidase of step (c) to hydroxylapatite chromatography; and

(e) recovering any methionine-specific aminopeptidase purified by said hydroxylapatite chromatography, and, especially,

(f) subjecting said recovered methionine-specific aminopeptidase of step (e) to additional ion exchange chromatography; and

(g) recovering any methionine-specific aminopeptidase purified by said additional ion exchange chromatography.

2. The method of claim1,characterized in that said sample is isolated from yeast growing at mid-log or late-log phase.

3. The process of claim 1 or 2, characterized by isolating a substantially purified methionine-specific aminopeptidase enzyme having an ability to cleave the peptide bond between an amino-terminal methionine residue, and every penultimate amino-terminal amino acid residue selected from the group consisting of: Ala, Asp, Glu, Gln, Gly, Ile, Leu, Lys, Met, Phe, Ser and Thr.

4. The process of claim 1 or 2, characterized by isolating a substantially purified methionine-specific aminopeptidase enzyme having an ability to cleave the peptide bond between an amino-terminal methionine residue, and every penultimate amino-terminal amino acid residue selected from the group consisting of: Val, Ala, Asp, Glu, Gln, Gly, His, Pro, Ser and Thr, and having an ability to cleave the peptide bond between an amino-terminal residue X, and a penultimate Ile residue of a peptide, wherein X is an amino-terminal amino acid residue selected from the group consisting of: Leu, Phe and Trp.

5. A process for the production of the methionine-specific aminopeptidases MAS I or MAS X, characterized by expressing a recombinant DNA molecule coding for the said aminopeptidase in a suitable host cell.

6. A method for removing an amino-terminal methionine residue from a peptide containing said amino-terminal residue, and containing as a penultimate amino-terminal amino acid residue, an amino acid selected from the group consisting of Ala, Asp, Glu, Gln, Gly, Ile, Leu, Lys, Met, Phe, Ser and Thr, characterized by incubating said peptide in the presence of the methionine-specific aminopeptidase MAS I to thereby remove said amino-terminal residue.

7. A method for removing an amino-terminal methionine residue from a peptide containing said amino-terminal residue, and containing as a penultimate amino-terminal amino acid residue, an amino acid selected from the group consisting of: Val, Ala, Asp, Glu, Gln, Gly, His, Pro, Ser and Thr, characterized by incubating said peptide in the presence of the methionine-specific aminopeptidase MAS X to thereby remove said amino-terminal residue.

8. A method for removing an amino-terminal residue X selected from the group consisting of: Leu, Phe and Trp from a peptide containing said aminoterminal residue, and containing as a penultimate amino-terminal amino residue Ile, characterized by incubating said peptide in the presence of the aminopeptidase MAS X to thereby remove said amino-terminal residue X.

9. The method of claim 7, 8 or 9, characterized in that said methionine-specific aminopeptidase is immobilized to a solid support.

15